# EUROPEAN PATENT APPLICATION

(11) **EP 4 442 219 A1**
(43) Date of publication of application: **09.10.2024**
(21) Application number: 22897684.1
(22) Date of filing: 17.11.2022
(51) Int. Cl.: A61B 34/30, A61B 34/20, B25J 9/10

(54) **MECHANICAL ZERO POSITION CALIBRATION METHOD AND APPARATUS, AND DEVICE AND MEDIUM**

(30) Priority: 29.11.2021 CN 202111437553
(71) Applicant: Ronovo (Shanghai) Medical Science and Technology Ltd., Shanghai 201102 (CN)
(72) Inventor: PAN, Le, Shanghai 201102 (CN); SUN, Xiaowen, Shanghai 201102 (CN); MAO, Ying, Shanghai 201102 (CN)
(74) Representative: dompatent von Kreisler Selting Werner - Partnerschaft von Patent- und Rechtsanwälten mbB
(86) International application number: PCT/CN2022/132485
(87) International publication number: WO 2023/093601

(57) **Abstract**

Disclosed in the embodiments of the present application are a mechanical zero position calibration method and apparatus, and a device and a medium. The method comprises: controlling a target electric motor to drive an instrument joint of a target instrument to respectively move in at least two set directions, and determining joint torque change information corresponding to each set direction; determining critical point information, corresponding to each set direction, of the instrument joint on the basis of the joint torque change information corresponding to each set direction; and determining, according to the critical point information corresponding to each set direction, an electric motor position corresponding to a mechanical zero position of the target instrument.

## Description

The present application claims the priority of Chinese patent application No. 202111437553.X filed on November 29, 2021. The contents of the Chinese patent application are incorporated herein by reference in their entireties.

### TECHNICAL FIELD

These embodiments of the present application relates to the technical field of surgical robots, for example, to a mechanical zero position calibration method and apparatus, and device and medium.

### BACKGROUND

In robotic surgery, surgical instruments usually need to determine the mechanical zero position before they can perform movements with absolute angles (such as pitching, yaw, or opening and closing). For example, mechanical zero position means that when defining the angle of an instrument joint, it is necessary to define a certain mechanical state of the instrument joint as zero degrees. Zero calibration refers to obtaining the motor angle corresponding to the drive motor when the instrument is at the mechanical zero position.

Therefore, the accuracy of the mechanical zero position determines the control accuracy of the joint angle of the target instrument. Due to the small size of surgical instruments, it is difficult to add more mechanical marks. Related technologies usually use some concave and convex alignment mechanisms to achieve calibration. However, due to limitations in application scenarios, it is usually impossible to use external professional equipment for on-site calibration, such as cleanliness requirements, space limitations and the need for professionals, etc. Related technologies often only calibrate a machinery once when the machinery is produced and shipped from the factory.

Because the drive chain of surgical instruments often have poor stiffness and are prone to deformation, and in the N+1 drive structure, the degree of freedom of the drive input is 1 more than the degree of freedom of the output, often resulting in the zero position of surgical instruments deviates, after they leaving the factory, and undergo operations like storage, transportation, handling, etc, leading to the decrease of movement accuracy during actual operations, thus affecting the effectiveness of the surgical operation.

### CONTENT OF THE PRESENT INVENTION

Disclosed in these embodiments of the present application are a mechanical zero position calibration method and apparatus, and device and medium, to avoid the situation that surgical instruments can not be zero position calibrated readily.

In a first aspect, disclosed in this embodiment of the present application is a mechanical zero position calibration method, and the method comprises:
controlling a target electric motor to drive an instrument joint of a target instrument to respectively move in at least two set directions, and determining joint torque change information corresponding to each set direction;
determining critical point information, corresponding to each set direction, of the instrument j oint on the basis of the joint torque change information corresponding to each set direction;
determining, according to the critical point information corresponding to each set direction, an electric motor position corresponding to a mechanical zero position of the target instrument.

In a second aspect, disclosed in this embodiment of the present application is a mechanical zero position calibration apparatus, and the apparatus comprises:
a torque change determination module, configured to control a target electric motor to drive an instrument joint of a target instrument to respectively move in at least two set directions, and determine joint torque change information corresponding to each set direction;
a critical point determination module, configured to determine critical point information, corresponding to each set direction, of the instrument joint on the basis of the joint torque change information corresponding to each set direction;
a position determination module, configured to determine, according to the critical point information corresponding to each set direction, an electric motor position corresponding to a mechanical zero position of the target instrument.

In a third aspect, disclosed in this embodiment of the present application is a electronic device, and the electronic device comprises:
one or more processors;
a storage device, configured to store one or more programs,
when the one or more programs are executed by the one or more processors, the one or more processors are caused to implement the mechanical zero position calibration method which is disclosed in any embodiment of the present application.

In a fourth aspect, disclosed in this embodiment of the present application is a computer-readable storage medium storing a computer program, when the program is executed by a processor, implementing the mechanical zero position calibration method which is disclosed in any embodiment of the present application.

### BRIEF DESCRIPTION OF THE DRAWINGS

Fig. 1A is a schematic flow chart of a mechanical zero position calibration method which is disclosed in an embodiment of the present application.
Fig. 1B is a schematic structural diagram of the target instrument which is disclosed in an embodiment of the present application.
Fig. 1C is a schematic diagram of the critical point which is disclosed in an embodiment of the present application.
Fig. 1D is a schematic diagram of a torque command relationship curve which is disclosed in an embodiment of the present application.
Fig. 2A is a schematic flow chart of a mechanical zero position calibration method which is disclosed in another embodiment of the present application.
Fig. 2B is a schematic diagram of the target instrument move in pitching direction or the yaw direction disclosed in an embodiment of the present application.
Fig. 3 is a schematic flow chart of a mechanical zero position calibration method which is disclosed in another embodiment of the present application.
Fig. 4 is a schematic structural diagram of a mechanical zero position calibration apparatus which is disclosed in an embodiment of the present application.
Fig. 5 is a schematic structural diagram of electronic device which is disclosed in an embodiment of the present application.

### DETAILED DESCRIPTION OF THE PREFERRED EMBODIMENT

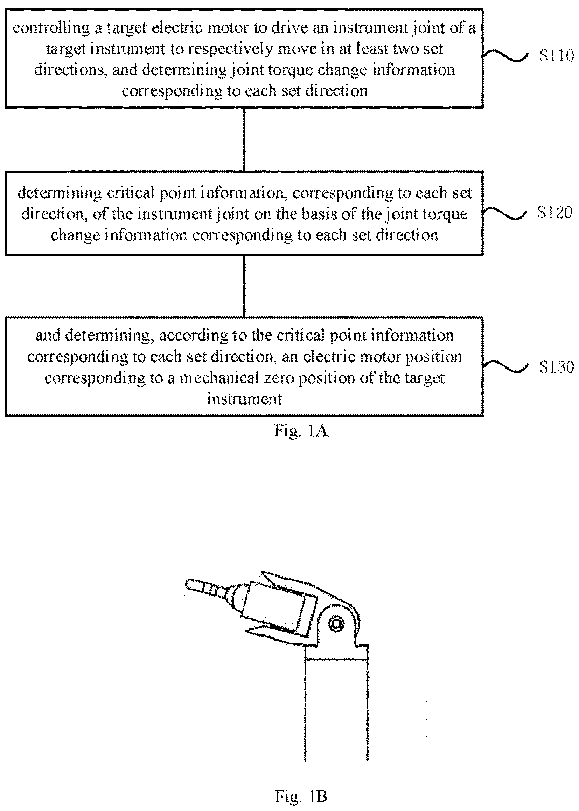

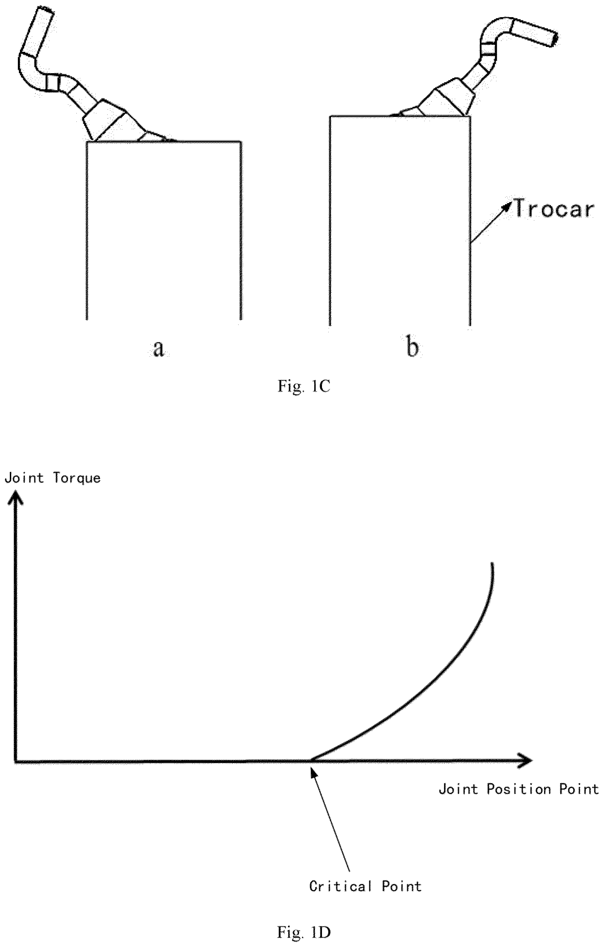

Fig. 1A is a schematic flow chart of a mechanical zero position calibration method which is disclosed in an embodiment of the present application. Disclosed in this embodiment can be applied to the automatic calibration of a mechanical zero position. For example, it is suitable for controlling an electric motor of an instrument to drive instrument joints to move in a plurality of set directions, and performing mechanical zero position calibration based on the joint torque change information obtained from the movement. The method can be performed by a mechanical zero calibration device, which can be implemented by hardware and/or software. The method comprises the following steps:

S 110. controlling a target electric motor to drive an instrument joint of a target instrument to respectively move in at least two set directions, and determining joint torque change information corresponding to each set direction.

Disclosed in this embodiment, the target instrument may be a surgical instrument. As shown in Fig. 1B, a schematic structural diagram of the target instrument is shown. Wherein, the target electric motor can drive the instrument joint of the target instrument to move, for example, the number of target electric motor can be one or more, the number of instrument joints can be one or more.

For example, the method disclosed in this embodiment can automatically control the target electric motor, to drive instrument joints to move in a plurality of set directions respectively. In this embodiment, the mechanical zero position calibration of the target instrument can include at least one of yaw angle zero position calibration, pitching angle zero position calibration and opening angle zero position calibration. For any one of yaw angle zero position calibration, pitching angle zero position calibration and opening angle zero position calibration requires driving the instrument joint to move in two set directions respectively. Exemplarily, taking the yaw angle zero position calibration as an example, the set directions include two yaw directions with opposite directions; taking the pitching angle zero position calibration and the opening angle zero position calibration as an example, then the set directions include two pitching directions with opposite directions, and moving to a first opening angle, moving from the first opening angle to a second opening angle.

It should be noted that the target electric motor can drive the instrument joint of the target instrument to move in a set direction in a relative constraint relationship until the target instrument touches the inner wall of the trocar, or reaches the j oint limit of the target instrument itself; and, obtaining the joint torque change information. Of course, every time drive the instrument joint of the target instrument to move in a set direction, the joint torque change information corresponding to this set direction can be obtained. Therefore, this embodiment can obtain joint torque change information corresponding to a plurality of set directions based on the above method. Wherein, joint torque change information may be information that the joint torque changes with changes in the joint position instructions. Exemplary, the joint position command may be a command that includes an angular magnitude of the movement of the instrument joint in a set direction. That is, the joint torque change information may include information that the joint torque of instrument joint changes with the changes of the angle of the movement.

S 120. determining critical point information, corresponding to each set direction, of the instrument joint on the basis of the joint torque change information corresponding to each set direction;

Wherein, the critical point information may be the mechanically restricted point of the instrument joint in the set direction. Exemplarily, the critical points include a contact point between the target instrument and an inner wall of the trocar, a contact point between the target instrument and a cannula outlet of the trocar, and a mechanical limit position point of the instrument joint. Exemplarily, as shown in Fig. 1C, showing a schematic diagram of a critical point, for example, in a and b of Fig. 1C, the critical point is a contact point between the target instrument and a cannula outlet of the trocar. Referring to Fig. 1B, the critical point may also be a mechanical limit position point of the instrument joint of the target instrument. In this embodiment, when the instrument joint of the target instrument moves to a critical point along the set direction, the value of the joint torque at that moment increases from zero. For example, the critical point information may be an actual motor position corresponding to the critical point, that is, the actual motor position corresponding to the moment when the value of the joint torque increases from zero.

For example, regardless of the influence of friction, the joint torque is always close to zero before the instrument joint moves in the set direction to the critical point; at the moment when the instrument j oint moves in the set direction to the critical point, the j oint torque begins to increase, and, after the instrument joint moves along the set direction to the critical point, due to the elastic characteristics of the target instrument drive rope, the relationship between the joint torque and the movement angle is a monotonic growth. Therefore, in this embodiment, the critical point may be determined based on the moment when the joint torque increases from zero in the joint torque change information. In this way, determining the critical point information corresponding to the plurality of set directions respectively.

In an exemplary embodiment, the determining critical point information, corresponding to each set direction, of the instrument joint on the basis of the joint torque change information corresponding to each set direction, comprising: based on the torque change information corresponding to each set direction, determining a torque command relationship curve corresponding to each set direction, wherein, the horizontal axis of the torque command relationship curve is a joint position command, the vertical axis of the torque command relationship curve is the joint torque; determining a target joint position command based on the torque command relationship curve, determining the critical point information corresponding to each set direction of the instrument joint based on the electric motor reference position corresponding to the target joint position command.

For example, the corresponding joint position command and the joint torque may be collected in a set period, obtaining the corresponding joint position command and joint torque in each period, that is, joint torque change information. For example, performing polynomial fitting on joint position commands and joint torques at multiple moments by the least squares method, and drew a torque command relationship curve which horizontal axis is the joint position command and which vertical axis is the joint torque. Exemplarily, as shown in FIG. 1D, taking the setting direction as a yaw direction or a pitch direction as an example, showing a diagram of a torque command relationship curve.

For example, after determine the torque command relationship curve, the joint position command corresponding to the moment when the joint torque increases from zero in the torque command relationship curve is determined as the target joint position command, and the actual motor position corresponding to the target joint position command, that is, the motor reference position, is determined as the critical point information corresponding to the set direction. Of course, it is also possible to find the intersection point between the curve and the joint position command axis in the torque command relationship curve, obtaining the displacement information of the critical point, recording the motor reference position corresponding to the intersection point, and determining it as the critical point information. By this exemplary embodiment, for each set direction, which can determine the critical point information corresponding to the set direction based on the collected joint torque change information, realizing accurate determination of the critical point information corresponding to the set direction, thereby improving the accuracy of mechanical zero position calibration.

S130. determining, according to the critical point information corresponding to each set direction, an electric motor position corresponding to a mechanical zero position of the target instrument.

In this embodiment, the critical point information corresponding to the plurality of set directions can be separately determined by the above method. The determining, according to the critical point information corresponding to each set direction, an electric motor position corresponding to a mechanical zero position of the target instrument which may be: determining the median value of the critical point informations corresponding to the two opposite set directions as the electric motor position corresponding to the mechanical zero position of the target instrument.

Exemplarily, taking the set direction as including two opposite pitching directions as an example, since the relative position of the target instrument and the cannula is limited by the mechanical structure, the target instrument is located in the center of the cannula, and the electric motor position corresponding to the mechanical zero position of the target instrument is(P1+P2)/2, wherein P1 and P2 are the critical point information corresponding to the two opposite pitching directions respectively, that is the actual position of the electric motor. Of course, the set directions may also include any two of two opposite pitching directions, two opposite yaw directions, and two opposite opening angle directions, taking the set directions including two opposite pitch directions and two opposite yaw directions as an example, the electric motor position corresponding to the mechanical zero position of the target instrument can be determined based on the two opposite pitching directions, the electric motor position may be mechanical zero position of one or more target electric motor that drive the instrument joints to move in the pitching directions; and determining the electric motor position corresponding to the mechanical zero position of the target instrument based on the two opposite yaw directions, the electric motor position may be mechanical zero position of one or more target electric motor that drive the instrument joints to move in the yaw directions.

It should be noted that the method disclosed in this embodiment can also reduce the impact of instrument backlash on zero calibration. For example, friction and the stiffness of the drive rope have a significant impact on the instrument transmission system, so the backlash is more significant on rope-driven instrument than on traditional system. The specific performance is that when the electric motor attempts to reverse, the joints have an angular lag in the reverse direction, it can also be described as: the electric motor has a range of motion, within this range of motion, the instrument joints will not move, there is no relationship between the electric motor and the instrument joints at this moment. Therefore, performing a traditional zero position calibration in this state will generate greater randomness and errors. The method disclosed in this embodiment, since during the calibration of the zero position of the electric motor corresponding to the mechanical zero position, the electric motor is under a stressed state close to one angle of one end, the electric motor is on both sides of the backlash, so the position of the electric motor is determined, which can provide the consistency of zero position calibration to a certain extent, eliminate the influence caused by randomness, and also provide certain conditions for backlash compensation.

The technical solution of this embodiment, by controlling a target electric motor to drive an instrument joint of a target instrument to respectively move in at least two set directions, to determine joint torque change information corresponding to each set direction; and determining the critical point information of the instrument joint corresponding to each set direction, on the basis of the joint torque change information corresponding to each set direction; and determining, according to the critical point information corresponding to each set direction, an electric motor position corresponding to the mechanical zero position of the target instrument. This method realizes the target instrument's automatic calibration of mechanical zero position, this method is not limited by scenarios such as site, equipment, technicians, etc, users can adopt this method to perform specified calibration of mechanical zero position at any time according to actual needs, avoiding the solution of the inability of related technologies to perform zero positioning of surgical instruments at any time, improving the movement accuracy of the instrument.

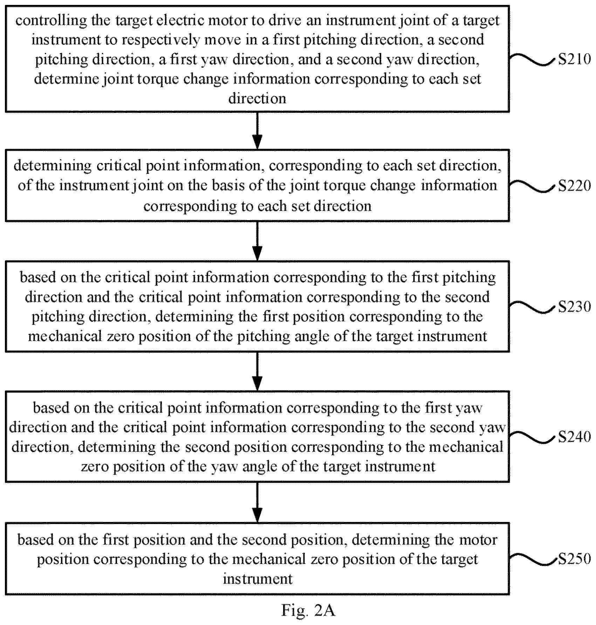

Fig. 2A is a schematic flow chart of a mechanical zero position calibration method which is disclosed in another embodiment of the present application. This embodiment is based on the above embodiment, for example, the controlling a target electric motor to drive an instrument joint of a target instrument to respectively move in at least two set directions, comprising: controlling a target electric motor to drive an instrument j oint of a target instrument to respectively move in a first pitching direction, a second pitching direction, a first yaw direction, and a second yaw direction, wherein, the first pitching direction is opposite to the second pitching direction, the first yaw direction is opposite to the second yaw direction. For example, the determining, according to the critical point information corresponding to each set direction, an electric motor position corresponding to a mechanical zero position of the target instrument, comprising: based on the critical point information corresponding to the first pitching direction and the critical point information corresponding to the second pitching direction, determine the first position corresponding to the mechanical zero position of the pitching angle of the target instrument; based on the critical point information corresponding to the first yaw direction and the critical point information corresponding to the second yaw direction, determining the second position corresponding to the mechanical zero position of the yaw angle of the target instrument; based on the first position and the second position, determining the motor position corresponding to the mechanical zero position of the target instrument. The explanations of terms that are same as or corresponding to the above multiple embodiments will not be repeated here. Referring to Fig.2A, the mechanical zero position calibration method disclosed in this embodiment comprises:

S210. controlling the target electric motor to drive an instrument joint of a target instrument to respectively move in a first pitching direction, a second pitching direction, a first yaw direction, and a second yaw direction, determining joint torque change information corresponding to each set direction.

Wherein, the first pitching direction is opposite to the second pitching direction, the first yaw direction is opposite to the second yaw direction. In this embodiment, by controlling of the electric motor to drive an instrument joint of a target instrument to respectively move in a first pitching direction, a second pitching direction, a first yaw direction, and a second yaw direction, obtaining joint torque change information corresponding to the first pitching direction, the second pitching direction, the first yaw direction, and the second yaw direction respectively.

S220. determining critical point information, corresponding to each set direction, of the instrument joint on the basis of the joint torque change information corresponding to each set direction.

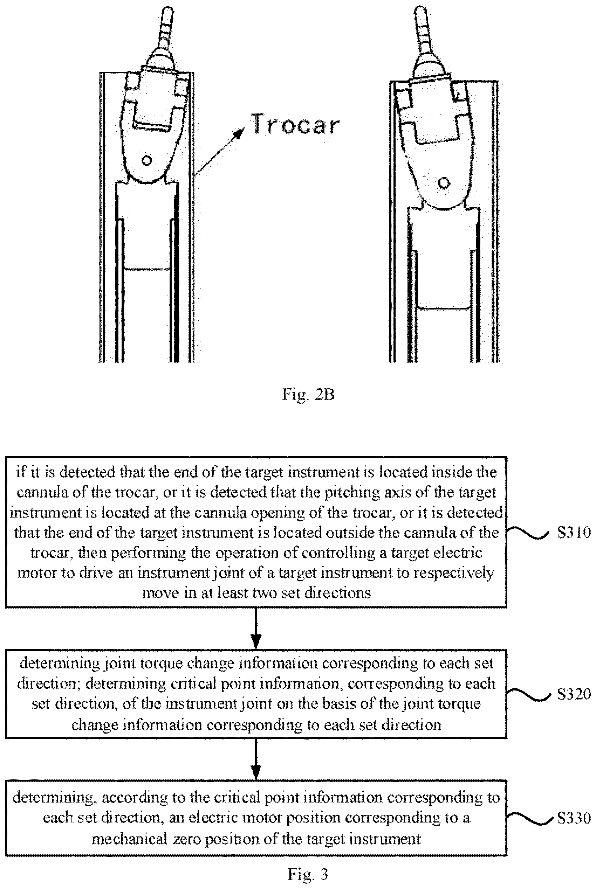

Wherein, the critical point information corresponding to each set direction can be determined based on the actual electric motor position corresponding to the moment when the joint torque increases from zero in the joint torque change information. Exemplarily, taking the critical point is a contact point between the target instrument and an inner wall of a trocar, as shown in Fig. 2B, showing a schematic diagram of the target instrument move in pitching direction or the yaw direction.

In this embodiment, it can determine the critical point information corresponding to the first pitching direction of the instrument joint based on the joint torque change information in the first pitching direction; determining the critical point information corresponding to the second pitching direction of the instrument joint based on the joint torque change information in the second pitching direction; determining the critical point information corresponding to the first yaw direction of the instrument j oint based on the j oint torque change information in the first yaw direction; determining the critical point information corresponding to the second yaw direction of the instrument joint based on the joint torque change information in the second yaw direction. Wherein, the critical point information can be the actual electric motor position corresponding to the critical point; the critical point can be a contact point between the target instrument and an inner wall of a trocar, a contact point between the target instrument and a cannula outlet of the trocar, or a mechanical limit position point of the instrument joint, the critical point can be determined based on the moment when the joint torque increases from zero.

S230. based on the critical point information corresponding to the first pitching direction and the critical point information corresponding to the second pitching direction, determining the first position corresponding to the mechanical zero position of the pitching angle of the target instrument.

Wherein, the mechanical zero position of the pitching angle may be the mechanical zero position of the target electric motor that drives the instrument joint to move along the first pitching direction and the second pitching direction. The first position corresponding to the mechanical zero position of the pitching angle of the target instrument may be an intermediate value between the critical point information corresponding to the first pitching direction and the critical point information corresponding to the second pitching direction.

S240. based on the critical point information corresponding to the first yaw direction and the critical point information corresponding to the second yaw direction, determining the second position corresponding to the mechanical zero position of the yaw angle of the target instrument.

Wherein, the mechanical zero position of the yaw angle may be the mechanical zero position of the target electric motor that drives the instrument joint to move in the first yaw direction and the second yaw direction. The first position corresponding to the mechanical zero position of the yaw angle of the target instrument may be an intermediate value between the critical point information corresponding to the first yaw direction and the critical point information corresponding to the second yaw direction.

S250. based on the first position and the second position, determine the motor position corresponding to the mechanical zero position of the target instrument.

In an exemplary embodiment, considering that some surgical instruments have opening angles, such as surgical scissors, the method disclosed in this embodiment comprises performing mechanical zero calibration on the opening angle direction of the target instrument. It should be noted that this embodiment can perform mechanical zero calibration on the opening angle direction, the pitching angle direction and the yaw angle direction at the same time, it can also perform mechanical zero position calibration only on the opening angle direction. That is, the controlling a target electric motor to drive an instrument j oint of a target instrument to respectively move in at least two set directions, comprising: controlling the target electric motor to drive the opening angle of the instrument joint of the target instrument to move to a first opening angle; controlling the target electric motor to drive the opening angle of the instrument joint of the target instrument to move from the first opening angle to a second opening angle; wherein, the first opening angle is greater than the second opening angle.

Exemplarily, the first opening angle may be 30°, and the second opening angle may be 0°. In this exemplary embodiment, it can first move the opening angle of the instrument joint of the target instrument to a larger angle, then reduce the opening angle of the instrument joint; during this process, when the opening angle of the instrument joint does not reach 0°, the value of the j oint torque is zero, when the opening angle of the instrument j oint is 0°, that is, in a merged state, the value of the joint torque starts to increase from zero, if still continuing to reduce the opening angle of the instrument joint at this time, the clamping force of the opening angle will increase monotonically.

For example, it can collect the joint torques and joint position commands corresponding to multiple moments within a set period, obtaining the joint torque change information when the opening angle moves to the first opening angle, and the opening angle moves from the first opening angle to the second opening angle, performing polynomial fitting on multiple points of the joint torque change information, obtaining the torque command relationship curve. The electric motor reference position corresponding to the intersection point of the torque command relationship curve and the horizontal axis (target joint position command) is determined as the critical point information corresponding to the opening angle direction. By this exemplary embodiment, it can realize the mechanical zero calibration of the opening angle.

The technical solution of this embodiment, by controlling a target electric motor to drive an instrument joint of a target instrument to respectively move in a first pitching direction, a second pitching direction, a first yaw direction, and a second yaw direction, to based on the critical point information corresponding to the first pitching direction and the critical point information corresponding to the second pitching direction, determining the first position corresponding to the mechanical zero position of the pitching angle of the target instrument, and based on the critical point information corresponding to the first yaw direction and the critical point information corresponding to the second yaw direction, determining the second position corresponding to the mechanical zero position of the yaw angle of the target instrument, further according to the first position and the second position, determining the motor position corresponding to the mechanical zero position of the target instrument, realizing the automatic calibration of the pitching angle mechanical zero position and the yaw angle mechanical zero position of the target instrument, this method is not limited by scenarios such as site, equipment, technicians, etc, users can adopt this method to perform specified calibration of mechanical zero position at any time according to actual needs, avoiding the solution of the inability of related technologies to perform zero positioning of surgical instruments at any time, improving the movement accuracy of the instrument.

Fig. 3 is a schematic flow chart of a mechanical zero position calibration method which is disclosed in another embodiment of the present application. This embodiment is based on the above embodiments, for example, if it is detected that the end of the target instrument is located inside the cannula of the trocar, or it is detected that the pitching axis of the target instrument is located at the cannula opening of the trocar, or it is detected that the end of the target instrument is located outside the cannula of the trocar, then performing the operation of controlling a target electric motor to drive an instrument j oint of a target instrument to respectively move in at least two set directions. The explanations of terms that are same as or corresponding to the above multiple embodiments will not be repeated here. Referring to Fig. 3, the mechanical zero calibration method disclosed in this embodiment comprises:

S310. if it is detected that the end of the target instrument is located inside the cannula of the trocar, or it is detected that the pitching axis of the target instrument is located at the cannula opening of the trocar, or it is detected that the end of the target instrument is located outside the cannula of the trocar, then performing the operation of controlling a target electric motor to drive an instrument joint of a target instrument to respectively move in at least two set directions.

In this embodiment, before performing the operation of controlling a target electric motor to drive an instrument joint of a target instrument to respectively move in at least two set directions, the surgical instrument needs to be loaded into the target instrument. For example, since most surgical instruments are consumables and need to be replaced frequently, the surgical instrument transmission chain and the electric motor output flange cannot be connected when leaving factories, and users need to install them on site in the operating room. After the installation of the surgical instrument is completed, the end of the target instrument should located inside the cannula of the trocar, or the pitching axis of the target instrument should located at the cannula opening of the trocar, or the end of the target instrument should located outside the cannula of the trocar.

For example, in this embodiment, if it is detected that the end of the target instrument is located inside the cannula of the trocar, or it is detected that the pitching axis of the target instrument is located at the cannula opening of the trocar, or it is detected that the end of the target instrument is located outside the cannula of the trocar, assuring the installation of the surgical instrument is completed, then performing the operation of controlling a target electric motor to drive an instrument joint of a target instrument to respectively move in at least two set directions.

For example, considering that after the installation of the instrument, the electric motor is required to load the tension of the drive rope while the target instrument maintains its current position, so as to avoid the target instrument to contact the inner wall of the trocar in advance and thus interfering with subsequent calibration work, therefore, before the controlling a target electric motor to drive an instrument joint of a target instrument to respectively move in at least two set directions, the method further comprises: loading the tension of the drive rope component based on a preset rope constraint condition.

Wherein, the rope constraint condition may be 1+D2+D3......+Dn=0, D is the stroke of the drive rope, n represents the driving degree of freedom of the driving rope. For example, after loading the tension of the driving rope component, the operation of controlling the target electric motor to drive the instrument joint of a target instrument to respectively move in at least two set directions can be continued, by this exemplary embodiment, early contact between the target instrument and the inner wall of the trocar can be avoided, thereby interfering with subsequent calibration work.

S320. determining joint torque change information corresponding to each set direction; determining critical point information, corresponding to each set direction, of the instrument joint on the basis of the joint torque change information corresponding to each set direction.

S330. determining, according to the critical point information corresponding to each set direction, an electric motor position corresponding to a mechanical zero position of the target instrument.

For example, a joint torque obtaining method also disclosed in this embodiment, that is, the method further comprises: determining a joint torque during a process of the instrument joint moves in the set direction; the determining a joint torque during a process of the instrument joint moves in the set direction, comprising: determining a joint torque during a process of the instrument joint moves in the set direction, based on the instrument force mapping matrix of the target instrument, the drive rope tension of the target instrument, and the drive rope friction of the target instrument.

Exemplarily, Joint Torque = Instrument Force Mapping Matrix x (Drive Rope Tension - Drive Rope Friction). Wherein, the drive rope tension = drive motor torque/drive pulley radius; the drive rope friction force is determined by the dynamic friction model of the rope and the pulley groove in the pulley system, and the instrument force mapping matrix is defined by the specific instrument drive structure. By this exemplary embodiment, the joint torque of the instrument joint during the movement in multiple set directions can be accurately determined, and the joint torque change information can be accurately determined based on the joint torque collected at multiple moments, thereby improving the calibration accuracy of the mechanical zero position.

The technical solution of this embodiment, when it is detected that the end of the target instrument is located inside the cannula of the trocar, or that the pitching axis of the target instrument is located at the cannula opening of the trocar, or that the end of the target instrument is located outside the cannula of the trocar, then performing the operation of controlling a target electric motor to drive an instrument joint of a target instrument to respectively move in at least two set directions, realizing automatic calibration of the mechanical zero position of the target instrument when it is automatically determined that the instrument loading is completed, avoiding the solution of the inability of related technologies to perform zero position calibration of surgical instruments at any time, improving the movement accuracy of the instrument.

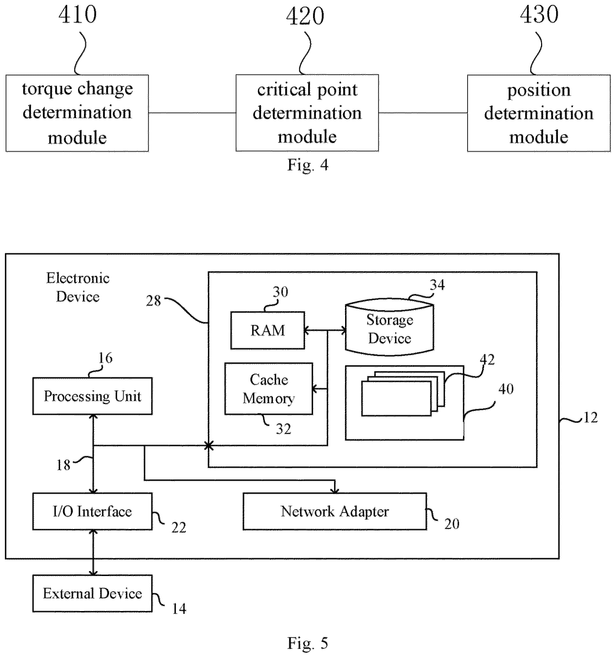

Fig. 4 is a schematic structural diagram of a mechanical zero position calibration apparatus which is disclosed in an embodiment of the present application, disclosed in this embodiment can be applied to the automatic calibration of a mechanical zero position. For example, it is suitable for controlling an electric motor of an instrument to drive instrument joints to move in a plurality of set directions, and performing mechanical zero position calibration based on the joint torque change information obtained from the movement, this apparatus comprises: a torque change determination module 410, a critical point determination module 420, and a position determination module 430.

A torque change determination module 410, configured to control a target electric motor to drive an instrument joint of a target instrument to respectively move in at least two set directions, and determine joint torque change information corresponding to each set direction;

A critical point determination module 420, configured to determine critical point information, corresponding to each set direction, of the instrument joint on the basis of the joint torque change information corresponding to each set direction;

A position determination module 430, configured to determine, according to the critical point information corresponding to each set direction, an electric motor position corresponding to a mechanical zero position of the target instrument.

For example, the torque change determination module 410 includes a joint control movement unit, the joint control movement unit is configured to control a target electric motor to drive an instrument joint of a target instrument to respectively move in a first pitching direction, a second pitching direction, a first yaw direction, and a second yaw direction, wherein, the first pitching direction is opposite to the second pitching direction, the first yaw direction is opposite to the second yaw direction.

For example, the position determination module 430 is configured to:

based on the critical point information corresponding to the first pitching direction and the critical point information corresponding to the second pitching direction, determine the first position corresponding to the mechanical zero position of the pitching angle of the target instrument; based on the critical point information corresponding to the first yaw direction and the critical point information corresponding to the second yaw direction, determine the second position corresponding to the mechanical zero position of the yaw angle of the target instrument; based on the first position and the second position, determine the motor position corresponding to the mechanical zero position of the target instrument.

For example, the joint control motion unit is further configured to control the target electric motor to drive the opening angle of the instrument joint of the target instrument to move to the first opening angle; control the target electric motor to drive the opening angle of the instrument joint of the target instrument to move from the first opening angle to the second opening angle; wherein, the first opening angle is greater than the second opening angle.

For example, the critical point determination module 420 is configured to:
based on the torque change information corresponding to each set direction, determine a torque command relationship curve corresponding to each set direction, wherein, the horizontal axis of the torque command relationship curve is a joint position command, the vertical axis of the torque command relationship curve is the joint torque; determine a target joint position command based on the torque command relationship curve, based on the electric reference position corresponding to the target joint position determine the critical point information corresponding to each set direction of the instrument joint.

For example, the apparatus further includes a joint torque determination module, the joint torque determination module is configured to determine a joint torque during a process of the instrument joint moves in the set direction; the joint torque determination module is configured to: determine a joint torque during a process of the instrument joint moves in the set direction, based on the instrument force mapping matrix of the target instrument, the drive rope tension of the target instrument, and the drive rope friction of the target instrument.

For example, the critical points include a contact point between the target instrument and an inner wall of a trocar, a contact point between the target instrument and a cannula outlet of the trocar, and a mechanical limit position point of the instrument joint.

For example, the apparatus further includes execution determination module, which is configured to if it is detected that the end of the target instrument is located inside the cannula of the trocar, or it is detected that the pitching axis of the target instrument is located at the cannula opening of the trocar, or it is detected that the end of the target instrument is located outside the cannula of the trocar, then perform the operation of controlling a target electric motor to drive an instrument joint of a target instrument to respectively move in at least two set directions.

For example, the apparatus further includes a tension loading module, and the tension loading module is configured to before the control a target electric motor to drive an instrument joint of a target instrument to respectively move in at least two set directions, the method further comprises: load the tension of the drive rope component based on a preset rope constraint condition.

In this embodiment, by the torque change determination module, control a target electric motor to drive an instrument joint of a target instrument to respectively move in at least two set directions, and determine joint torque change information corresponding to each set direction; further by the critical point determination module, determine critical point information, corresponding to each set direction, of the instrument joint on the basis of the joint torque change information corresponding to each set direction; by the position determination module, determine, according to the critical point information corresponding to each set direction, an electric motor position corresponding to a mechanical zero position of the target instrument, this method realizes the target instrument's automatic calibration of mechanical zero position, this method is not limited by scenarios such as site, equipment, technicians, etc, users can adopt this method to perform specified calibration of mechanical zero position at any time according to actual needs, avoiding the solution of the inability of related technologies to perform zero position calibration of surgical instruments at any time, improving the movement accuracy of the instrument.

The mechanical zero position calibration apparatus disclosed in this embodiment of the present application can execute the mechanical zero position calibration methods provided by any embodiment of the present application, and has functional modules and beneficial effects corresponding to the execution method.

It is worth noting that the multiple units and modules included in the above system are only divided according to functional logic, but are not limited to the above divisions, as long as they can achieve the corresponding functions; in addition, the specific names of the multiple functional units are only used to facilitate mutual differentiation and are not used to limit the protection scope of the embodiments of the present application.

Fig. 5 is a schematic structural diagram of electronic device which is disclosed in an embodiment of the present application. Fig. 5 shows a block diagram of an exemplary electronic device 12 suitable for implementing the embodiment of the present application. The electronic device 12 shown in Fig. 5 is only an example and should not bring any limitations to the functions and scope of use of the embodiments of the present application. Device 12 is typically an electronic device that performs the function of determining the mechanical zero position calibration.

As shown in Fig. 5, the electronic device 12 is represented in the form of a general computing device. The components of the electronic device 12 may include, but are not limited to: one or more processors or processing units 16, a memory 28, and a bus 18 connecting different components (including the memory 28 and the processing unit 16).

Bus 18 represents one or more types of bus structures, including a memory bus or a memory controller, a peripheral bus, a graphics accelerated port, a processor, or a local bus using any of a variety of bus structures. For example, these architectures include but are not limited to the Industry Standard Architecture (ISA) bus, the Micro Channel Architecture (MCA) bus, the enhanced ISA bus, the Video Electronics Standards Association (VESA) local bus, and Peripheral Component Interconnect (PCI) bus.

Electronic device 12 typically includes a variety of computer-readable medium. These medium may be any available medium that can be accessed by electronic device 12, including volatile and non-volatile medium, removable and non-removable medium.

Memory 28 may include computer device readable medium in the form of volatile memory, such as random access memory (RAM) 30 and/or cache memory 32. Electronic device 12 may include other removable/non-removable, volatile/non-volatile computer storage medium. By way of example only, storage device 34 may be used to read and write from non-removable, non-volatile magnetic medium (not shown in Fig. 5, commonly referred to as "hard drive"). Although not shown in Fig. 5, a disk drive may be provided for reading and writing to a removable non-volatile disk (such as "floppy disk"), and a disk drive for reading and writing to a removable non-volatile optical disk (such as a Compact Disc- Read Only Memory (CD-ROM), Digital Video Disc (Digital Video Disc-Read Only Memory, DVD-ROM) or other optical medium) reading and writing optical disc drive. In these cases, each drive may be connected to bus 18 through one or more data medium interfaces. Memory 28 may include at least one program product 40 having a set of program modules 42, these program modules are configured to perform the functions of multiple embodiments of the present application. Program product 40 may be stored, for example, in memory 28. Such program modules 42 include, but are not limited to, one or more application programs, other program modules, and program data. Each of these examples, or some combination of these examples, may include a realization of network environment. Program modules 42 generally perform functions and/or methods in the embodiments described in this application.

Electronic device 12 may also communicate with one or more external devices 14 (for example, keyboard, mouse, camera, etc. and display), may also communicate with one or more devices that enable a user to interact with electronic device 12, and/or with any device (for example, network card, modem, etc.) that enables the electronic device 12 to communicate with one or more other computing devices. This communication may occur through input/output (I/O) interface 22. And, the electronic device 12 may also communicate with one or more networks (for example, Local Area Network (LAN), Wide Area Network (WAN), and/or a public network, such as the Internet) by means of the network adapter 20. As shown in the figure, network adapter 20 communicates with other modules of electronic device 12 via bus 18. It should be understood that, although not shown in the figures, other hardware and/or software modules may be used in conjunction with the electronic device 12, including but not limited to: microcode, device drivers, redundant processing units, external disk drive arrays, disk arrays (Redundant Arrays of Independent Disks, RAID) devices, tape drives, and data backup storage devices, etc.

The processing unit 16 executes a variety of functional applications and data processing by running programs stored in the memory 28, such as implementing the mechanical zero position calibration method provided by the above embodiments of the present application, comprising:
controlling a target electric motor to drive an instrument joint of a target instrument to respectively move in at least two set directions, and determining joint torque change information corresponding to each set direction;
determining critical point information, corresponding to each set direction, of the instrument joint on the basis of the joint torque change information corresponding to each set direction;
and determining, according to the critical point information corresponding to each set direction, an electric motor position corresponding to a mechanical zero position of the target instrument.

Of course, those skilled in the art can understand that the processor can also implement the technical solution of the mechanical zero position calibration method provided by any embodiment of the present application.

Embodiments of the present application also provide a computer-readable storage medium on which a computer program is stored, when the program is executed by a processor, the steps of the mechanical zero position calibration method provided by any embodiment of the present application are implemented, the method comprises:
Controlling a target electric motor to drive an instrument joint of a target instrument to respectively move in at least two set directions, and determining joint torque change information corresponding to each set direction;
determining critical point information, corresponding to each set direction, of the instrument joint on the basis of the joint torque change information corresponding to each set direction;

and determining, according to the critical point information corresponding to each set direction, an electric motor position corresponding to a mechanical zero position of the target instrument.

The computer storage medium in the embodiment of the present application may be any combination of one or more computer-readable medium. The computer-readable medium may be a computer-readable signal medium or a computer-readable storage medium. The computer-readable storage medium may be, for example, but is not limited to: an electrical, magnetic, optical, electromagnetic, infrared, or semiconductor system, apparatus or device, or any combination of the above. More specific examples (non-exhaustive list) of computer-readable storage medium include: electrical connections having one or more conductors, portable computer disks, hard drives, random access memory (RAM), read only memory (ROM), Erasable programmable read-only memory (EPROM or flash memory), fiber optics, portable compact disk read-only memory (CD-ROM), optical storage devices, magnetic storage devices, or any suitable combination of the above. In this document, the computer-readable storage medium may be any tangible medium that contains or stores a program for use by or in connection with an instruction execution system, apparatus, or device. The computer-readable storage medium may be a non-transitory computer-readable storage medium.

A computer-readable signal medium may comprise a data signal propagated in baseband or as part of a carrier wave carrying computer-readable program code therein. Such propagated data signals may take many forms, including but not limited to electromagnetic signals, optical signals, or any suitable combination of the above. The computer-readable signal medium may also be any computer-readable medium other than a computer-readable storage medium that can send, propagate, or transmit a program for use by or in conjunction with an instruction execution system, apparatus, or device.

Program code embodied on the computer-readable medium may be transmitted using any suitable medium, including but not limited to wireless, wire, optical cable, RF, etc., or any suitable combination of the above.

Computer program code for performing operations of embodiments of the present application may be written in one or more programming languages, including object-oriented programming languages - such as Java, Smalltalk, C++, and combinations thereof, including Conventional procedural programming language - such as "C" or similar programming language. The program code may execute entirely on the user's computer, partly on the user's computer, as a stand-alone software package, partly on the user's computer and partly on a remote computer or entirely on the remote computer or server. In situations involving remote computers, the remote computer can be connected to the user's computer through any kind of network, including a local area network (LAN) or a wide area network (WAN), or it can be connected to an external computer (such as an Internet service provider by Internet connection).

## Claims

1. A mechanical zero position calibration method, comprising:
controlling a target electric motor to drive an instrument joint of a target instrument to respectively move in at least two set directions, and determining joint torque change information corresponding to each set direction;
determining critical point information, corresponding to each set direction, of the instrument joint on the basis of the joint torque change information corresponding to each set direction;
and determining, according to the critical point information corresponding to each set direction, an electric motor position corresponding to a mechanical zero position of the target instrument.

2. The method as claimed in claim 1, wherein, the controlling a target electric motor to drive an instrument joint of a target instrument to respectively move in at least two set directions, comprising:
controlling the target electric motor to drive the instrument joint of the target instrument to respectively move in a first pitching direction, a second pitching direction, a first yaw direction, and a second yaw direction, wherein, the first pitching direction is opposite to the second pitching direction, the first yaw direction is opposite to the second yaw direction.

3. The method as claimed in claim 2, wherein, the determining, according to the critical point information corresponding to each set direction, an electric motor position corresponding to a mechanical zero position of the target instrument, comprising:
based on the critical point information corresponding to the first pitching direction and the critical point information corresponding to the second pitching direction, determining a first position corresponding to the mechanical zero position of pitching angle of the target instrument;
based on the critical point information corresponding to the first yaw direction and the critical point information corresponding to the second yaw direction, determining a second position corresponding to the mechanical zero position of yaw angle of the target instrument;
based on the first position and the second position, determining the motor position corresponding to the mechanical zero position of the target instrument.

4. The method as claimed in claim 1 or 2, wherein, the controlling a target electric motor to drive an instrument joint of a target instrument to respectively move in at least two set directions, comprising:
controlling the target electric motor to drive opening angle of the instrument joint of the target instrument to move to a first opening angle;
controlling the target electric motor to drive the opening angle of the instrument joint of the target instrument to move from the first opening angle to a second opening angle;
wherein, the first opening angle is greater than the second opening angle.

5. The method as claimed in claim 1, wherein, the determining critical point information, corresponding to each set direction, of the instrument joint on the basis of the joint torque change information corresponding to each set direction, comprising:
based on the torque change information corresponding to each set direction, determining a torque command relationship curve corresponding to each set direction, wherein, horizontal axis of the torque command relationship curve is a joint position command, vertical axis of the torque command relationship curve is a joint torque;
determining a target joint position command based on the torque command relationship curve, based on an electric reference position corresponding to the target joint position command, determining the critical point information corresponding to each set direction of the instrument joint.

6. The method as claimed in claim 1, further comprising: determining a j oint torque during a process of the instrument joint moves in the set direction;
the determining a joint torque during a process of the instrument joint moves in the set direction, comprising:
determining the joint torque during the process of the instrument joint moves in the set direction, based on an instrument force mapping matrix of the target instrument, a drive rope tension of the target instrument, and a drive rope friction of the target instrument.

7. The method as claimed in claim 1, for example, the critical points include a contact point between the target instrument and an inner wall of a trocar, a contact point between the target instrument and a cannula outlet of the trocar, and a mechanical limit position point of the instrument joint.

8. The method as claimed in claim 1, further comprising:
in response to detecting that the position of the target instrument satisfies one of the following conditions, performing an operation of controlling the target electric motor to drive the instrument joint of the target instrument to respectively move in at least two set directions:
an end of the target instrument is located inside the cannula of the trocar; a pitching axis of the target instrument is located at the cannula opening of the trocar; and an end of the target instrument is located outside the cannula of the trocar.

9. The method as claimed in claim 8, before the controlling the target electric motor to drive the instrument joint of the target instrument to respectively move in at least two set directions, the method further comprises:
loading a tension of a drive rope component based on a preset rope constraint condition.

10. A mechanical zero position calibration apparatus, comprising:
a torque change determination module, configured to control a target electric motor to drive an instrument joint of a target instrument to respectively move in at least two set directions, and determine a joint torque change information corresponding to each set direction;
a critical point determination module, configured to determine critical point information, corresponding to each set direction, of the instrument joint on the basis of the joint torque change information corresponding to each set direction;
a position determination module, configured to determine, according to the critical point information corresponding to each set direction, an electric motor position corresponding to a mechanical zero position of the target instrument.

11. An electric device, comprising:
one or more processors;
a storage device, configured to store one or more programs,
when the one or more programs are executed by the one or more processors, the one or more processors are caused to implement the mechanical zero position calibration method as claimed in any one of claims 1-9.

12. A computer-readable storage medium, storing a computer program, when the computer program is executed by a processor, implementing the mechanical zero position calibration method as claimed in any one of claims 1-9.
